(19) Europäisches Patentamt
European Patent Office
Office européen des brevets

(11) Veröffentlichungsnummer: **0 424 849 B1**

(12) **EUROPÄISCHE PATENTSCHRIFT**

(45) Veröffentlichungstag der Patentschrift: **13.04.94**

(51) Int. Cl.[5]: **C07D 239/52**, C07D 239/56, C07D 239/58

(21) Anmeldenummer: **90120239.0**

(22) Anmeldetag: **22.10.90**

Die Akte enthält technische Angaben, die nach dem Eingang der Anmeldung eingereicht wurden und die nicht in dieser Patentschrift enthalten sind.

# (54) Verfahren zur Herstellung von Aminopyrimidinen.

(30) Priorität: **24.10.89 DE 3935278**

(43) Veröffentlichungstag der Anmeldung:
**02.05.91 Patentblatt 91/18**

(45) Bekanntmachung des Hinweises auf die Patenterteilung:
**13.04.94 Patentblatt 94/15**

(84) Benannte Vertragsstaaten:
**AT BE CH DE FR GB IT LI NL**

(56) Entgegenhaltungen:
**EP-A- 0 024 200**

**CHEMICAL ABSTRACTS, Band 103, 1985, Seite 613, Zusammenfassung Nr. 87900k, Columbus, Ohio, US**

(73) Patentinhaber: **HOECHST AKTIENGESELLSCHAFT**

**D-65926 Frankfurt(DE)**

(72) Erfinder: **Lachhein, Stephen, Dr.**
**Kiedricher Strasse 18**
**W-6238 Hofheim am Taunus(DE)**
Erfinder: **Willms, Lothar, Dr.**
**Lindenstrasse 17**
**W-5416 Hillscheid(DE)**

EP 0 424 849 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Gegenstand der Erfindung ist ein Verfahren zur Herstellung von Pyrimidinen der Formel I

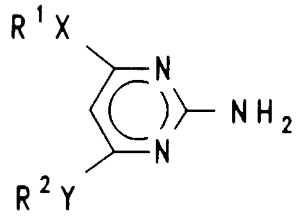

I

worin
X und Y jeweils Sauerstoff oder Schwefel und
$R^1$, $R^2$ = unabhängig voneinander $(C_1\text{-}C_4)$Alkyl, $(C_1\text{-}C_4)$Alkoxy-$(C_1\text{-}C_2)$alkyl oder Halo$(C_1\text{-}C_4)$alkyl bedeutet,
aus einem Propandiimidat der Formel II

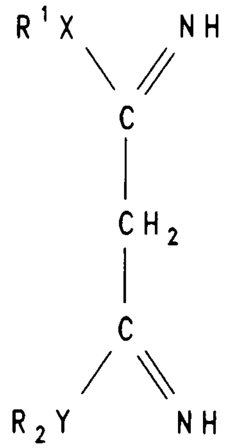

II

oder dessen Salz, wobei $R^1$, $R^2$, X und Y die bei Formel I definierte Bedeutung haben, in Anwesenheit eines Alkohols und eines inerten Lösungsmittels, dadurch gekennzeichnet, daß man die Verbindung der Formel II mit einem Salz des Cyanamides der Formel III

$(R^3)_j\ (H^+)_k\ [N\text{-}C \equiv N]^{2-}$ III

worin
$R^3$ ein Alkalimetallkation bedeutet und
j = 1 und k = 1 oder
j = 2 und k = 0
bedeuten, umsetzt, wobei eine Lösung der Verbindungen der Formeln II und III in einem Alkohol der Formel IV

$R^4$-OH IV

worin
$R^4$ $(C_1\text{-}C_6)$-Alkyl bedeutet,
oder getrennte Lösungen der Verbindungen der Formeln II und III in jeweils einem Alkohol der genannten Formel IV simultan in ein im Vergleich zu dem Alkohol höhersiedendes inertes organisches Lösungsmittel bei Temperaturen oberhalb von 100 °C unter gleichzeitigem Abdestillieren des Alkohols der Formel IV gegeben werden.

Die Verbindungen der Formel I sind wertvolle Zwischenprodukte bei der Herstellung von Sulfonylharnstoffen mit herbizider Wirkung; s. z. B. US 4,169,719 oder EP 071 958 (US-A-4,492,598).

In den Resten $R^1$ und $R^2$ bedeutet Alkyl Methyl, Ethyl, Propyl, Isopropyl, n-, i-, t- oder 2-Butyl. Beim Rest $R^4$ kommen dazu noch die geradkettigen oder verzweigten Pentyle oder Hexyle in Frage. Haloalkyl bedeutet durch Fluor, Chlor, Brom oder Jod substituiertes Alkyl. Alkoxy bedeutet Alkyl-oxy mit den im Alkylteil genannten möglichen Bedeutungen.

Bekannt ist, daß Pyrimidine der Formel I durch Umsetzung von Propanidiimidaten mit wäßriger Cyanamidlösung oder Chlorcyan in einem Zwei- oder Dreistufenprozeß hergestellt werden können (EP-A-0 024 200), wobei die Bildung und anschließende Isolierung eines N-Cyanoimidates als Zwischenprodukt als charakteristische Verfahrensmerkmale anzusehen sind. Die hierbei erhältlichen Ausbeuten für das Endprodukt sind jedoch unbefriedigend. Weitere Nachteile des genannten Verfahrens sind: die Bildung nicht unerheblicher Mengen an Nebenprodukten, die Notwendigkeit der Reaktionsführung in einem engen pH-Bereich und die zusätzliche Verwendung einer Base. Außerdem ist es nachteilig, daß die während der Umsetzung gebildeten anorganischen Salze, speziell beim Einsatz von wäßriger Cyanamidlösung, in gelöster Form ins Abwasser gelangen, aus welchem sie durch zusätzliche Maßnahmen entfernt werden müssen.

Überraschenderweise stellt das erfindungsgemäße Verfahren demgegenüber einen verfahrenstechnisch einfachen Einstufenprozeß ohne isolierbare Zwischenprodukte dar, der eine Reaktionsführung ohne pH-Kontrolle gestattet. Nebenprodukte werden nur in untergeordnetem Maße gebildet.

Beim erfindungsgemäßen Verfahren greifen die stark basischen Salze des Cyanamids an der Imidatstruktur an. Die hohe Selektivität zur Bildung von Verbindungen der Formel I und die damit hohen Ausbeuten waren aufgrund der stark basischen Natur der Cyanamidsalze und der gegen Nucleophile empfindlichen Struktur der Propandiimidate nicht zu erwarten. Außerdem ist bekannt, daß Imidate oder Bisimidate besonders in ihrer Salzform thermolabil sind. Es war deshalb nicht zu erwarten, daß die Propandiimidate bei den hohen Reaktionstemperaturen oberhalb von 100 °C so stabil sind, daß hohe Ausbeuten von etwa 90 % und mehr beim erfindungsgemäßen Verfahren erhalten werden können.

Die Reaktion wird in einem nicht wäßrigen, unter den Reaktionsbedingungen inerten organischen Lösungsmittel durchgeführt, so daß praktisch kein freies Cyanamid durch Hydrolyse gebildet werden kann. Die während der Reaktion gebildeten anorganischen Salze fallen als Feststoffe aus, die abfiltriert werden und somit nicht in das Abwasser gelangen können. Neben den bereits beschriebenen verfahrenstechnischen und wirtschaftlichen Vorteilen ist dieser Aspekt des Verfahrens aus ökologischer Sicht als fortschrittlich anzusehen. Nach Beendigung der Reaktion und Abdestillieren des Lösungsmittels, welches nahezu quantitativ zurückgewonnen wird, verbleibt das Reaktionsprodukt in hoher Reinheit.

Das erfindungsgemäße Verfahren kann beispielsweise so durchgeführt werden, daß man das Propandiimidat als Monosalz, Disalz oder als freies Bisimidat in einem Alkohol, vorzugsweise bei -10 °C bis +10 °C, zusammen mit einem Cyanamidsalz der Formel III löst und die erhaltene Lösung in das inerte organische Lösungsmittel oberhalb von 100 °C eindosiert. Alternativ kann man beispielsweise eine auf -10 °C bis +10 °C temperierte Lösung von Propandiimidat als Monosalz, Disalz oder als freies Bisimidat in einem Alkohol simultan mit einer Lösung eines Cyanamidsalzes der Formel II in einem Alkohol in das inerte organische Lösungsmittel oberhalb von 100 °C eindosieren. Während der kontinuierlichen Zugabe der alkoholischen Lösung bzw. der alkoholischen Lösungen wird der Alkohol kontinuierlich aus dem Reaktionsgemisch abdestilliert.

Bei der Reaktion zwischen den Verbindungen der Formeln II und III lassen sich keine Zwischenverbindungen isolieren oder nachweisen, sondern es bilden sich direkt die Pyrimidine der Formel I.

Als Salze des Propandiimidates werden bevorzugt solche der Fluor-, Chlor- oder Bromwasserstoffsäure, der Schwefel- oder Phosphorsäure eingesetzt. Haloalkylreste für $R^1$, $R^2$ sind beispielsweise $CH_2CH_2Cl$ oder $CH_2CF_3$. Bevorzugte Bedeutung für X, Y ist Sauerstoff und für $R^1$, $R^2$ $(C_1\text{-}C_4)$Alkyl, insbesondere Methyl.

Die Wahl des inerten organischen Lösungsmittels hängt von dem Siedepunkt des Alkohols ab. Der Alkohol muß sich während der Umsetzung aus dem Reaktionsgemisch abdestillieren lassen. Lösungsmittel sind beispielsweise aliphatische und gegebenenfalls halogenierte aromatische Kohlenwasserstoffe, aber auch Lösungsmittel aus der Gruppe der Ketone, wie Methylisobutylketon, und anderer chemischer Klassen.

Als Alkohole kommen vor allem Methanol und Ethanol in Frage. Bevorzugt als inerte organische Lösungsmittel sind aromatische Kohlenwasserstoff wie z. B. Toluol, Xylol oder Chlorbenzol.

Um störende Einflüsse von Sauerstoff auf die Reaktion zu vermeiden, ist es zweckmäßig, unter Inertgasatmosphäre, beispielsweise unter Stickstoff, zu arbeiten.

Die Verbindungen der Formel II können nach bekannten Methoden hergestellt werden (S. M. McElvain und I. D. Schröder, JACS 71, 40 (1949); B. Harstun, DOS 2 426 913).

Nachfolgende Beispiele sollen das erfindungsgemäße Verfahren näher erläutern:

Beispiel 1:

In 1000 ml Methanol werden unter Stickstoffüberlagerung bei 0 °C 102 g Dimethylpropandiimidatdihydrochlorid und 33 g Mononatriumcyanamid gelöst und innerhalb von 2 Stunden in 120 °C heißes Chlorbenzol getropft. Während dieser Zeit wird kontinuierlich das Methanol abdestilliert und reichert sich das Produkt in der Chlorbenzolphase an. Nach beendeter Reaktion wird vom ausgefallenen Salz abfiltriert und das Chlorbenzol abdestilliert. Es verbleiben 69,3 g 2-Amino-4,6-dimethoxypyrimidin, was einer Ausbeute von 89 % der Theorie entspricht. Der Schmelzpunkt beträgt 92 bis 94 °C. Analoge Ergebnisse werden mit Toluol oder Xylol als inertem Lösungsmittel erhalten.

Beispiel 2:

In 500 ml Ethanol werden unter Stickstoff bei + 10 °C 102 g Dimethylpropandiimidatdihydrochlorid gelöst und simultan mit einer Lösung von 43 g Dinatriumcyanamid in 500 ml Ethanol in 120 °C heißes Chlorbenzol innerhalb von 3 Stunden eindosiert. Während dieser Zeit wird das Ethanol kontinuierlich abdestilliert. Nach beendeter Reaktion wird von ausgefallenen Salz abfiltriert und das Chlorbenzol abdestilliert. Es verbleiben 70,0 g 2-Amino-4,6-dimethoxypyrimidin, was einer Ausbeute von 90 % der Theorie entspricht. Der Schmelzpunkt beträgt 92 bis 93 °C. Analoge Ergebnisse werden mit Toluol und Xylol als inertem Lösungsmittel erhalten.

Entsprechend den in Beispielen 1 oder 2 beschriebenen Verfahrensweisen werden beispielsweise folgende Verbindungen der Formel I erhalten:

| Beispiel | X | Y | $R^1$ | $R^2$ |
|---|---|---|---|---|
| 3 | O | O | $C_2H_5$ | $C_2H_5$ |
| 4 | O | O | $CH(CH_3)-CH_3$ | $CH(CH_3)-CH_3$ |
| 5 | S | S | $CH_3$ | $CH_3$ |
| 6 | S | S | $C_2H_5$ | $C_2H_5$ |
| 7 | S | S | $CH(CH_3)-CH_3$ | $CH(CH_3)-CH_3$ |

**Patentansprüche**

1. Verfahren zur Herstellung von Verbindungen der Formel I

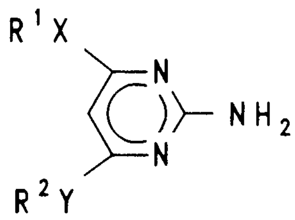

I,

worin
X und Y jeweils Sauerstoff oder Schwefel und
$R^1$, $R^2$ = unabhängig voneinander $(C_1\text{-}C_4)$Alkyl, $(C_1\text{-}C_4)$Alkoxy-$(C_1\text{-}C_2)$alkyl oder Halo$(C_1\text{-}C_4)$alkyl bedeutet,
aus einem Propandiimidat der Formel II

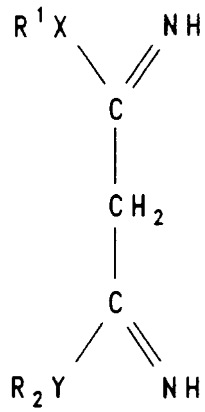

II

oder dessen Salz, wobei $R^1$, $R^2$, X und Y die bei Formel I definierte Bedeutung haben, in Anwesenheit eines Alkohols und eines inerten Lösungsmittels, dadurch gekennzeichnet, daß man die Verbindung der Formel II mit einem Salz des Cyanamides der Formel III

$(R^3)_j\ (H^+)_k\ [N\text{-}C \equiv N]^{2-}$ III,

worin
$R^3$ ein Alkalimetallkation bedeutet und
j = 1 und k = 1 oder
j = 2 und k = 0
bedeuten, umsetzt, wobei eine Lösung der Verbindungen der Formeln II und III in einem Alkohol der Formel IV

$R^4$-OH IV,

worin
$R^4$ $(C_1\text{-}C_6)$-Alkyl bedeutet,
oder getrennte Lösungen der Verbindungen der Formeln II und III in jeweils einem Alkohol der genannten Formel IV simultan in ein im Vergleich zu dem Alkohol höhersiedendes inertes organisches Lösungsmittel bei Temperaturen oberhalb von 100 °C unter gleichzeitigem Abdestillieren des Alkohols der Formel IV gegeben werden.

2. Verfahren gemäß Anspruch 1, dadurch gekennzeichnet, daß in Formel I
X und Y jeweils ein Sauerstoffatom und
$R^1$ und $R^2$ $(C_1-C_4)$Alkyl bedeuten.

3. Verfahren gemäß Anspruch 1 und 2, dadurch gekennzeichnet, daß in Formel I $R^1$ und $R^2$ jeweils Methyl oder Ethyl bedeuten.

4. Verfahren gemäß einem oder mehreren der Ansprüche 1 bis 3, dadurch gekennzeichnet, daß man als Salze der Verbindung der Formel II solche der Fluor-, Chlor- oder Bromwasserstoffsäure, der Schwefel- oder Phosphorsäure verwendet.

5. Verfahren nach einem oder mehreren der Ansprüche 1 bis 4, dadurch gekennzeichnet, daß man als inertes organisches Lösungsmittel einen gegebenenfalls halogenierten Kohlenwasserstoff einsetzt.

6. Verfahren nach Anspruch 5, dadurch gekennzeichnet, daß Chlorbenzol, Toluol oder Xylol eingesetzt wird.

7. Verfahren nach einem oder mehreren der Ansprüche 1 bis 6, dadurch gekennzeichnet, daß der Alkohol der Formel IV Methanol oder Ethanol ist.

8. Verfahren nach einem oder mehreren der Ansprüche 1 bis 7, dadurch gekennzeichnet, daß die Lösung bzw. die Lösungen der Verbindungen der Formel II oder III im Alkohol der Formel IV auf -10 bis +10 °C temperiert sind.

9. Verfahren nach einem oder mehreren der Ansprüche 1 bis 8, dadurch gekennzeichnet, daß die Reaktionslösung nach der Reaktion von ausgefallenen anorganischen Salzen abfiltriert und das inerte organische Lösungsmittel abdestilliert wird.

10. Verfahren nach Anspruch 5, dadurch gekennzeichnet, daß Chlorbenzol als inertes organisches Lösungsmittel eingesetzt wird.

**Claims**

1. A process for the preparation of compounds of the formula I

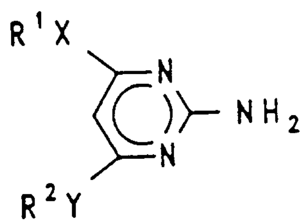

I,

in which
X and Y are in each case oxygen or sulfur and
$R^1$, $R^2$ are independently of one another $(C_1-C_4)$alkyl, $(C_1-C_4)$alkoxy-$(C_1-C_2)$alkyl or halo$(C_1-C_4)$-alkyl,
from a propanediimidate of the formula II

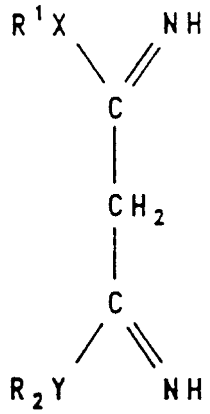

II

or its salt, where $R^1$, $R^2$, X and Y have the meaning defined for formula I, in the presence of an alcohol and an inert solvent which comprises reacting the compound of the formula II with a salt of the cyanamide of the formula III

$(R^3)_j(H^+)_k[N\text{-}C{\equiv}N]^{2-}$ III,

in which
$R^3$ is an alkali metal cation and
j = 1 and k = 1 or
j = 2 and k = 0,
in which a solution of the compounds of the formulae II and III in an alcohol of the formula IV

$R^4$-OH IV,

in which
$R^4$ is $(C_1\text{-}C_6)$alkyl,
or, simultaneously, separate solutions of the compounds of the formulae II and III in each case in an alcohol of said formula IV are added to an inert organic solvent which is higher-boiling compared to the alcohol at temperatures above 100 °C with simultaneous removal of the alcohol of the formula IV by distillation.

**2.** The process as claimed in claim 1, wherein, in formula I,
X and Y are in each case an oxygen atom and
$R^1$ and $R^2$ are $(C_1\text{-}C_4)$alkyl.

**3.** The process as claimed in claim 1 and 2, wherein, in formula I, $R^1$ and $R^2$ are in each case methyl or ethyl.

**4.** The process as claimed in one or more of claims 1 to 3, wherein salts of the compound of the formula II which are used are those of hydrofluoric, hydrochloric or hydrobromic acid, sulfuric acid or phosphoric acid.

**5.** The process as claimed in one or more of claims 1 to 4, wherein an optionally halogenated hydrocarbon is employed as the inert organic solvent.

**6.** The process as claimed in claim 5, wherein chlorobenzene, toluene or xylene is employed.

**7.** The process as claimed in one or more of claims 1 to 6, wherein the alcohol of the formula IV is methanol or ethanol.

**8.** The process as claimed in one or more of claims 1 to 7, wherein the solution or the solutions of the compounds of the formula II or III in the alcohol of the formula IV are adjusted to -10 to +10 °C.

**9.** The process as claimed in one or more of claims 1 to 8, wherein precipitated inorganic salts are filtered off from the reaction solution after the reaction and the inert organic solvent is removed by distillation.

**10.** The process as claimed in claim 5, wherein chlorobenzene is employed as the inert organic solvent.

## Revendications

**1.** Procédé pour préparer des composés de formule I

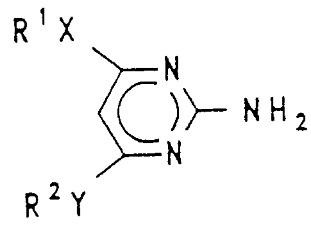

I,

où
X et Y représentent chacun l'oxygène ou le soufre et
$R^1$, $R^2$ représentent, de façon indépendante l'un de l'autre, un alkyle en $C_1$-$C_4$, un alcoxy en $C_1$-$C_4$-alkyle en $C_1$-$C_2$ ou un halogénoalkyle en $C_1$-$C_4$,
à partir d'un propanediimidate de formule II

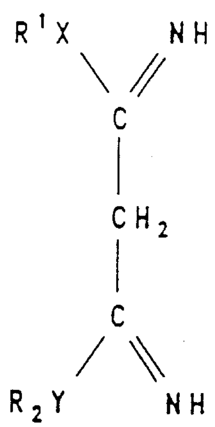

II

ou ses sels, où $R^1$, $R^2$, X et Y ont la signification donnée pour la formule I, en présence d'un alcool et d'un solvant inerte, le procédé étant caractérisé en ce que l'on fait réagir le composé de formule II avec un sel du cyanamide de formule III

$(R^3)_j\ (H^+)_k\ [N\text{-}C \equiv N]^{2-}$ III,

où
$R^3$ représente un cation de métal alcalin et,
$j = 1$ et $k = 1$ ou
$j = 2$ et $k = 0$
où une solution des composés des formules II et III dans un alcool de formule IV

$R^4$-OH IV,

où
$R^4$ représente un alkyle en $C_1$-$C_6$,
ou des solutions séparées des composés des formules II et III, chaque fois dans un alcool de formule

donnée IV, simultanément, sont introduites dans un solvant organique, inerte dont le point d'ébullition est supérieur à celui de l'alcool, à des températures supérieures à 100° C, tout en chassant en même temps par distillation l'alcool de formule IV.

**2.** Procédé selon la revendication 1, caractérisé en ce que dans la formule I
X et Y représentent chaque fois un atome d'oxygène et
$R^1$ et $R^2$ représentent un alkyle en $C_1$-$C_4$.

**3.** Procédé selon la revendication 1 ou 2, caractérisé en ce que dans la formule I, $R^1$ et $R^2$ représentent chaque fois le méthyle ou l'éthyle.

**4.** Procédé selon une ou plusieurs des revendications 1 à 3, caractérisé en ce qu'on utilise en tant que sels du composé de formule II ceux des acides fluorhydrique, chlorhydrique ou bromhydrique, de l'acide sulfurique ou de l'acide phosphorique.

**5.** Procédé selon une ou plusieurs des revendications 1 à 4, caractérisé en ce qu'on utilise en tant que solvant organique inerte un hydrocarbone éventuellement halogéné.

**6.** Procédé selon la revendication 5, caractérisé en ce qu'on utilise le chlorobenzène, le toluène ou le xylène.

**7.** Procédé selon une ou plusieurs des revendications 1 à 6, caractérisé en ce que l'alcool de formule IV est le méthanol ou l'éthanol.

**8.** Procédé selon une ou plusieurs des revendications 1 à 7, caractérisé en ce que la solution ou les solutions des composés de formule II ou de formule III dans l'alcool de formule IV sont réglées à une température de -10 à +10° C.

**9.** Procédé selon une ou plusieurs des revendications 1 à 8, caractérisé en ce que l'on sépare par filtration la solution de réaction des sels minéraux précipités, après la réaction, et en ce que l'on chasse par distillation le solvant organique inerte.

**10.** Procédé selon la revendication 5, caractérisé en ce que l'on utilise le chlorobenzène comme solvant organique inerte.